# EUROPÄISCHE PATENTSCHRIFT

(11) **EP 0 799 814 B1**
(45) Veröffentlichungstag und Bekanntmachung des Hinweises auf die Patenterteilung: **20.09.2000**
(21) Anmeldenummer: 97101967.4
(22) Anmeldetag: 07.02.1997
(51) Int. Cl.: C07C 29/20, C07C 35/08, B01J 23/46

(54) **Verfahren zur Hydrierung von aromatischen Verbindungen, in denen mindestens eine Hydroxylgruppe an einen aromatischen Kern gebunden ist**
Method for the hydrogenation of aromatic compounds containing at least one hydroxyl group bound to an aromatic nucleus
Procédé pour la hydrogénation de composés aromatiques avec au moins un groupe hydroxyle lié à un noyau aromatique

(30) Priorität: 09.02.1996 DE 19604791
(43) Veröffentlichungstag der Anmeldung: 08.10.1997
(73) Patentinhaber: BASF AKTIENGESELLSCHAFT, 67056 Ludwigshafen (DE)
(72) Erfinder: Rütter, Heinz, Dr., 67126 Hochdorf-Assenheim (DE); Rühl, Thomas, Dr., 67227 Frankenthal (DE); Breitscheidel, Boris, Dr., 36043 Fulda (DE); Henkelmann, Jochem, Dr., 68165 Mannheim (DE); Wettling, Thomas, Dr., 67117 Limburgerhof (DE)
(74) Vertreter: Isenbruck, Günter, Dr.

(56) Entgegenhaltungen:
- EP-A- 0 147 751
- WO-A-97/10202
- DE-A- 2 458 587
- DE-A- 2 909 663
- FR-A- 2 023 832
- US-A- 4 424 162

## Beschreibung

Die vorliegende Erfindung betrifft ein Verfahren zur Hydrierung von aromatischen Verbindungen, in denen mindestens eine Hydroxylgruppe an einen aromatischen Kern gebunden ist.

Die vorliegende Erfindung betrifft insbesondere ein Verfahren zur Hydrierung von aromatischen Verbindungen, in denen zusätzlich zur mindestens einen Hydroxylgruppe mindestens ein, ggf. substituierter, C₁₋₁₀-Alkyl- und/oder Alkoxyrest an einen aromatischen Kern gebunden ist. Vorzugsweise werden Monoalkyl-substituierte Phenole im erfindungsgemäßen Verfahren eingesetzt. Die ein- oder mehrkernigen aromatischen Verbindungen werden dabei zu den entsprechenden cycloaliphatischen Verbindungen hydriert, wobei die Hydroxylgruppen erhalten bleiben, mit Hilfe von Katalysatoren, die Ruthenium und ggf. mindestens ein weiteres Metall der I-, VII- oder VIII-Nebengruppe auf einem Träger enthalten.

Cycloaliphatische Alkohole, insbesondere Alkylcyclohexanole, sind wichtige Zwischenprodukte für die Herstellung verschiedener Duftstoffe, Arzneimittel und anderer organischer Feinchemikalien.

Es ist bekannt, Alkylcyclohexanole durch katalytische Hydrierung der entsprechenden Alkylphenole herzustellen. Die Hydrierung von Alkylphenolen zu den entsprechenden Alkylcyclohexanolen in Gegenwart von Hydrierungskatalysatoren, insbesondere auf Trägern aufgebrachten Katalysatoren, ist vielfach beschrieben.

Als Katalysatoren wurden beispielsweise metallisches Rhodium, Ruthenium, Palladium sowie Nickel auf Katalysatorträgern verwendet. Als Katalysatorträger wurden Kohlenstoffe, Bariumcarbonat und insbesondere Aluminiumoxid eingesetzt.

In der PL 137 526 ist die Hydrierung von p-tert.-Butylphenol zu p-tert.-Butylcyclohexanol unter Verwendung eines Nickel-Katalysators beschrieben.

In der DE-A1-34 01 343 ist ein Verfahren zur Herstellung von 2- und 4-tert.-Butylcyclohexanol aus 2- und 4-tert.-Butylphenol durch katalytische Hydrierung beschrieben. Die Hydrierung wird zweistufig ausgeführt unter Verwendung in einer ersten Stufe eines Palladium-Katalysators auf einem Al₂O₃-Träger und in der zweiten Stufe eines Ruthenium-Katalysators auf einem Al₂O₃-Träger. Der Metallgehalt auf dem Träger betrug dabei 0,1 bis 5 Gew.-%. Die Träger sind nicht weiter spezifiziert. Es wurde bei einem Druck von 300 bar unter Produktruckführung gearbeitet. Es wurden vorzugsweise die cis-tert.-Butylphenole erhalten, wobei 0,1 bis 0,5% Nebenprodukte anfielen.

In der US 2 927 127 ist ein Verfahren zur Herstellung von p-tert.-Butylcyclohexanol und Estern daraus beschrieben durch katalytische Hydrierung von p-tert.-Butylphenol. Als Katalysatoren wurden 5% Rhodium auf Kohlenstoff, 5 % Palladium auf Bariumcarbonat oder 5% Ruthenium auf Kohlenstoff verwendet. Bei der Verwendung von Ruthenium auf Kohlenstoff wurde bei einem Druck von 70 bis 120 bar und einer Temperatur von 74 bis 93 °C gearbeitet. Als Hydrierungsprodukt wurden 66% cis-Isomer erhalten.

In der DE-A1-29 09 663 ist ein Verfahren zur Herstellung von cis-Alkylcyclohexanolen durch katalytische Hydrierung der entsprechenden Alkylphenole beschrieben. Als Katalysator wurde Ruthenium auf einem Al₂O₃-Träger verwendet, wobei der Träger eine spezifische Oberfläche im Bereich von 100 bis 300 m²/g und eine Korngrößenverteilung mit einer maximalen Korngröße von < 0,15 mm aufweisen soll. Es wurde bei Drücken von 40, 60 oder 80 bar gearbeitet. Als Produkt wurden überwiegend cis-Alkylcyclohexanole erhalten mit 0,1 bis 1% Alkylbenzol als Nebenprodukt.

US 4,424,162 betrifft ein Verfahren zur partiellen Hydrierung von eßbaren Ölen. Durch die Hydrierung soll die Stabilität der eßbaren Öle erhöht werden, jedoch soll die Bildung von Feststoffen vermieden werden. Insbesondere soll die Bildung von gesättigten und trans-Säuren bei der Hydrierung vermieden werden.

Als Katalysator für die Hydrierung wird Ruthenium auf Aluminiumoxid als Träger eingesetzt. Dabei kann die Oberfläche von α-Aluminiumoxid kleiner als 15m²/g sein. Zudem kann im Katalysator ein hoher Anteil an Makroporen vorliegen.

WO 97/10202 betrifft einen zur Durchführung von Hydrierverfahren geeigneten Katalysator aus Ruthenium und gegebenenfalls mindestens einem Metall der I-, VII- oder VIII-Nebengruppe, in einer Menge von 0,01 bis 30 Gew.-% aufgebracht auf einem Träger, wobei der Träger einen mittleren Porendurchmesser von mindestens 0,1 µm und eine Oberfläche von höchstens 15 m²/g aufweist.

DE-A-24 58 587 betrifft ein Verfahren zur Hydrodealkylierung von alkylaromatischen Kohlenwasserstoffen bei einer Temperatur von 400 bis 650°C unter einem Druck von 1 bis 30 kg/cm² bei einer Raumgeschwindigkeit von 1 bis 10. Der eingesetzte Katalysator enthält Aluminiumoxid und mindestens ein Metall ausgewählt aus Ruthenium, Osmium, Palladium, Rhodium, Iridium, Platin und Mangan und weist eine spezifische Oberfläche von 1 bis 100 m²/g bei einem Gesamtporenvolumen von 0,2 bis 0,8 ml/g auf.

Eine Aufgabe der vorliegenden Erfindung ist die Bereitstellung eines Verfahrens zur Hydrierung von Verbindungen, in denen mindestens eine Hydroxylgruppe und vorzugsweise mindestens ein gegebenenfalls substituierter C₁₋₁₀-Alkylrest und/oder Alkoxyrest an einen aromatischen Kern gebunden ist, zu den entsprechenden cycloaliphatischen Verbindungen mit sehr hoher Ausbeute bzw. nahezu vollständigem Umsatz.

Eine weitere Aufgabe der Erfindung ist die Bereitstellung eines Verfahrens zur Hydrierung von aromatischen Verbindungen, in denen mindestens eine Hydroxylgruppe und vorzugsweise mindestens ein gegebenenfalls substituierter C₁₋₁₀-Alkylrest und/oder Alkoxyrest an einen aromatischen Kern gebunden ist, zu den entsprechenden cycloaliphatischen Verbindungen, wobei nur ein minimaler Nebenproduktanteil bzw. Zersetzungsproduktanteil bei der Hydrierung anfällt.

Eine weitere Aufgabe der vorliegenden Erfindung ist die Bereitstellung eines Verfahrens zur Hydrierung von aromatischen Verbindungen, in denen mindestens eine Hydroxylgruppe und vorzugsweise mindestens ein gegebenenfalls substituierter C₁₋₁₀-Alkylrest und/oder Alkoxyrest an einen aromatischen Kern gebunden ist, wobei hohe Katalysatorbelastungen und lange Katalysatorstandzeiten ermöglicht werden.

Eine weitere Aufgabe der vorliegenden Erfindung ist die Bereitstellung eines einstufigen Verfahrens zur Hydrierung von aromatischen Verbindungen, in denen mindestens eine Hydroxylgruppe und vorzugsweise mindestens ein gegebenenfalls substituierter C₁₋₁₀-Alkylrest und/oder Alkoxyrest an einen aromatischen Kern gebunden ist.

Eine weitere Aufgabe der vorliegenden Erfindung ist die Bereitstellung eines Verfahrens zur Hydrierung von aromatischen Verbindungen, in denen mindestens eine Hydroxylgruppe und vorzugsweise mindestens ein gegebenenfalls substituierter C₁₋₁₀-Alkylrest und/oder Alkoxyrest an einen aromatischen Kern gebunden ist, wobei die erhaltenen cycloaliphatischen Verbindungen ohne weitere Reinigungsschritte weiterverarbeitet werden können.

Eine weitere Aufgabe der vorliegenden Erfindung ist die Bereitstellung eines Verfahrens zur Hydrierung von aromatischen Verbindungen, in denen mindestens eine Hydroxylgruppe und vorzugsweise mindestens ein ggf. substituierter C₁₋₁₀-Alkyl- und/oder Alkoxyrest an einen aromatischen Kern gebunden sind, zu den entsprechenden cycloaliphatischen Verbindungen, wobei überwiegend trans-cycloaliphatische Verbindungen entstehen.

Gelöst werden eine oder mehrere der vorstehenden Aufgaben durch ein Verfahren zur Hydrierung von aromatischen Verbindungen, in denen mindestens eine Hydroxylgruppe an einen aromatischen Kern gebunden ist, wobei man mindestens eine dieser Verbindungen in Gegenwart eines Katalysators mit freiem Wasserstoff in Berührung bringt, wobei der Katalysator Ruthenium und gegebenenfalls mindestens ein Metall der I-, VII- oder VIII-Nebengruppe in einer Menge von 0,01 bis 30 Gew.-%, vorzugsweise 0,2 bis 15 Gew.-% bezogen auf das Gesamtgewicht des Katalysators, aufgebracht auf einen Träger, umfaßt, wobei der Träger einen mittleren Porendurchmesser von mindestens 0,1 µm, und eine Oberfläche von höchstens 15 m²/g, vorzugsweise höchstens 10 m²/g, aufweist. Die vorstehenden Aufgaben sowie ggf. weitere Aufgaben werden gelöst durch Verfahren zur Hydrierung, wie sie im folgenden beschrieben sind.

Erfindungsgemäß wurde gefunden, daß statt der bekannten Hydrierungskatalysatoren, die typischerweise auf mesoporösen Trägern mit einer großen Oberfläche aufgebracht sind, um eine hohe Aktivität des Katalysators zu erzielen, Katalysatoren auf makroporösen Trägern verwendet werden können. Trotz der geringen Oberfläche der Katalysatoren, die Ruthenium und ggf. ein oder mehrere weitere Metalle der Nebengruppen I, VII oder VIII enthalten, können aromatische Verbindungen, in denen mindestens eine Hydroxylgruppe und vorzugsweise mindestens ein gegebenenfalls substituierter C₁₋₁₀-Alkylrest und/oder Alkoxyrest an einen aromatischen Kern gebunden ist, mit hoher Selektivität zu den entsprechenden cycloaliphatischen Alkoholen hydriert werden, wobei überwiegend trans-cycloaliphatische Verbindungen entstehen. Die Bildung von Nebenprodukten ist dabei beim erfindungsgemäßen Verfahren minimal. Insbesondere wird die Bildung von aromatischen Verbindungen, insbesondere Alkylaromaten bzw. Alkylbenzolen aus Alkylphenolen, praktisch vollständig vermieden.

Zudem können vorzugsweise hohe Katalysatorbelastungen und lange Katalysatorstandzeiten erzielt werden. Die Katalysatorbelastung ist dabei die Raum/Zeit-Ausbeute des Verfahrens, d.h. die Menge des umgesetzten Eduktes pro Zeiteinheit und pro Menge an vorliegendem Katalysator. Standzeit bedeutet die Zeit bzw. die Menge an umgesetztem Edukt, die ein Katalysator verkraftet, ohne seine Eigenschaften einzubüßen und ohne daß sich die Produkteigenschaften signifikant verändern.

### VERBINDUNGEN

Mit dem erfindungsgemäßen Verfahren können aromatische Verbindungen, in denen mindestens eine Hydroxylgruppe und vorzugsweise mindestens ein gegebenenfalls substituierter C₁₋₁₀-Alkylrest und/oder Alkoxyrest an einen aromatischen Kern gebunden ist, zu den entsprechenden cycloaliphatischen Verbindungen hydriert werden. Dabei können die aromatischen Verbindungen einkernige oder mehrkernige aromatische Verbindungen sein. Die aromatischen Verbindungen enthalten mindestens eine Hydroxylgruppe, die an einen aromatischen Kern gebunden ist, die einfachste Verbindung dieser Gruppe ist Phenol. Vorzugsweise weisen die aromatischen Verbindungen eine Hydroxylgruppe pro aromatischem Kern auf. Die aromatischen Verbindungen können an dem aromatischen Kern oder den aromatischen Kernen substituiert sein durch einen oder mehrere Alkyl- und/oder Alkoxyreste, vorzugsweise C₁₋₂₀-Alkyl- und/oder Alkoxyreste, besonders bevorzugt C₁₋₁₀-Alkylreste, insbesondere Methyl-, Ethyl-, Propyl-, Isopropyl-, Butyl-, Isobutyl-, tert.-Butylreste; unter den Alkoxyresten sind die C₁₋₈-Alkoxyreste bevorzugt. Der aromatische Kern oder die aromatischen Kerne sowie die Alkyl- und Alkoxyreste können gegebenenfalls durch Halogenatome, insbesondere Fluoratome, substituiert sein oder andere geeignete inerte Substituenten aufweisen.

Vorzugsweise weisen die erfindungsgemäß hydrierbaren Verbindungen mindestens einen, vorzugsweise einen bis vier, insbesondere einen C₁₋₂₀-Alkylrest auf, der sich vorzugsweise am gleichen aromatischen Kern befindet wie die mindestens eine Hydroxylgruppe. Bevorzugte Verbindungen sind (Mono)alkylphenole, wobei der Alkylrest in o-, m- oder p-Position zur Hydroxylgruppe stehen kann. Insbesondere bevorzugt sind trans-Alkylphenole, auch als 4-Alkylphenole bezeichnet, wobei der Alkylrest vorzugsweise 1 bis 10 Kohlenstoffatome aufweist und insbesondere ein tert.-Butylrest ist. Bevorzugte Verbindung ist 4-tert.-Butylphenol. Erfindungsgemäß verwendbare mehrkernige aromatische Verbindungen sind beispielsweise α-Naphthol, β-Naphthol.

Die aromatische Verbindung, in der mindestens eine Hydroxylgruppe und vorzugsweise mindestens ein gegebenenfalls substituierter C₁₋₁₀-Alkylrest und/oder Alkoxyrest an einen aromatischen Kern gebunden ist, kann auch mehrere aromatische Kerne aufweisen, die über einen Alkylenrest, vorzugsweise eine Methylengruppe, verknüpft sind. Die verknüpfende Alkylenkette, vorzugsweise Methylengruppe, kann einen oder mehrere Alkylsubstituenten aufweisen, die C₁₋₂₀-Alkylreste sein können, vorzugsweise C₁₋₁₀-Alkylreste, besonders bevorzugt Methyl, Ethyl-, Propyl-, Isopropyl-, Butyl-, sec.-Butyl- oder tert.-Butylreste sind.

Dabei kann jeder der aromatischen Kerne mindestens eine Hydroxylgruppe gebunden enthalten. Beispiele solcher Verbindungen sind Bisphenole, die in 4-Position über einen Alkylenrest, vorzugsweise einen Methylenrest, verknüpft sind. Ein bevorzugtes Beispiel ist Bisphenol A, in dem zwei Phenolmoleküle in der 4-Position über eine Dimethylmethylengruppe verknüpft sind.

### KATALYSATOREN

Die erfindungsgemäß verwendeten Katalysatoren können technisch hergestellt werden durch Auftragen von Ruthenium und gegebenenfalls mindestens einem Metall der I-, VII- oder VIII-Nebengruppe auf einen geeigneten Träger. Die Auftragung kann durch Tränken des Trägers in wäßrigen Metallsalzlösungen, wie Rutheniumsalzlösungen, durch Aufsprühen entsprechender Metallsalzlösungen auf den Träger oder durch andere geeignete Verfahren erreicht werden. Als Rutheniumsalze zur Herstellung der Rutheniumsalzlösungen wie auch als Metallsalze der I-, VII- oder VIII-Nebengruppe eignen sich die Nitrate, Nitrosylnitrate, Halogenide, Carbonate, Carboxylate, Acetylacetonate, Chlorkomplexe, Nitritokomplexe oder Aminkomplexe der entsprechenden Metalle, bevorzugt sind dabei die Nitrate und Nitrosylnitrate.

Bei Katalysatoren, die neben Ruthenium noch weitere Metalle auf den Träger aufgetragen enthalten, können die Metallsalze bzw. Metallsalzlösungen gleichzeitig oder nacheinander aufgebracht werden.

Die mit der Rutheniumsalz- bzw. Metallsalzlösung beschichteten bzw. getränkten Träger werden sodann getrocknet, vorzugsweise bei Temperaturen zwischen 100 °C und 150 °C, und wahlweise bei Temperaturen zwischen 200 °C und 600 °C calciniert.

Darauffolgend werden die beschichteten Träger aktiviert durch Behandlung der beschichteten Träger in einem Gasstrom, der freien Wasserstoff enthält, bei Temperaturen zwischen 30 und 600 °C, vorzugsweise zwischen 150 und 450 °C. Der Gasstrom besteht vorzugsweise aus 50 bis 100 Vol-% H₂ und 0 bis 50 Vol-% N₂.

Werden auf die Träger neben Ruthenium noch ein oder mehrere andere Metalle der I-, VII- oder VIII-Nebengruppe aufgetragen, und erfolgt das Auftragen nacheinander, so kann der Träger nach jedem Auftragen bzw. Tränken bei Temperaturen zwischen 100 und 150 °C getrocknet werden und wahlweise bei Temperaturen zwischen 200 und 600 °C calciniert werden. Dabei kann die Reihenfolge, in der die Metallsalzlösungen aufgetragen oder aufgetränkt werden, beliebig gewählt werden.

Werden neben Ruthenium ein oder mehrere weitere Metalle der I-, VII- oder VIII-Nebengruppe auf den Träger aufgebracht, so werden vorzugsweise Kupfer, Rhenium, Kobalt, Nickel oder deren Gemische verwendet.

Die Rutheniumsalzlösung bzw. Metallsalzlösung wird in einer solchen Menge auf den oder die Träger aufgebracht, daß 0,01 bis 30 Gew.-%, bezogen auf das Gesamtgewicht des Katalysators, an Ruthenium und gegebenenfalls anderem Metall oder anderen Metallen der I-, VII- oder VIII-Nebengruppe auf den Träger aufgebracht vorliegen. Vorzugsweise beträgt diese Menge 0,2 bis 15 Gew.-%, besonders bevorzugt etwa 0,5 Gew.-%.

Die Metalloberfläche auf dem Katalysator beträgt insgesamt vorzugsweise 0,01 bis 10 m²/g, besonders bevorzugt 0,05 bis 5 m²/g, insbesondere 0,05 bis 3 m² pro g des Katalysators.

### TRÄGER

Die zur Herstellung der erfindungsgemäß verwendeten Katalysatoren verwendbaren Trägermaterialien sind vorzugsweise solche, die makroporös sind und einen mittleren Porendurchmesser von mindestens 0,1 µm, vorzugsweise mindestens 0,5 µm, und eine Oberfläche von höchstens 15 m²/g aufweisen, vorzugsweise höchstens 10 m²/g, besonders bevorzugt höchstens 5 m²/g, insbesondere höchstens 3 m²/g. Bevorzugt liegt der mittlere Porendurchmesser des Trägers in einem Bereich von 0,1 bis 200 µm, insbesondere 0,5 bis 50 µm. Bevorzugt beträgt die Oberfläche des Trägers 0,2 bis 15 m²/g, besonders bevorzugt 0,5 bis 10 m²/g, insbesondere 0,5 bis 5 m²/g, speziell 0,5 bis 3 m² pro g des Trägers.

Die Oberfläche des Trägers wird bestimmt nach dem BET-Verfahren durch N₂-Adsorption, insbesondere nach DIN 66131. Die Bestimmung des mittleren Porendurchmessers und der Porengrößenverteilung erfolgte durch Hg-Porosimetrie, insbesondere nach DIN 66133. Vorzugsweise kann die Porengrößenverteilung des Trägers annähernd bimodal sein, wobei die Porendurchmesserverteilung mit Maxima bei etwa 0,6 µm und etwa 20 µm bei der bimodalen Verteilung eine spezielle Ausführungsform der Erfindung darstellt.

Besonders bevorzugt ist ein Träger mit einer Oberfläche von etwa 1,75 m²/g, der diese bimodale Verteilung des Porendurchmessers aufweist. Das Porenvolumen dieses bevorzugten Trägers beträgt vorzugsweise etwa 0,53 ml/g.

Als makroporöses Trägermaterial verwendbar sind beispielsweise Aktivkohle, Siliciumcarbid, Aluminiumoxid, Siliciumdioxid, Titandioxid, Zirkoniumdioxid, Magnesiumoxid, Zinkoxid oder deren Gemische. Bevorzugt sind Aluminiumoxid und Zirkoniumdioxid.

Die erfindungsgemäß verwendeten Katalysatoren zeigen vorzugsweise eine hohe Reaktivität, Selektivität und Standzeit. Unter Einsatz der erfindungsgemäß verwendeten Katalysatoren werden in der Hydrierung die Hydrierungsprodukte vorzugsweise in hoher Ausbeute und Reinheit gewonnen, wobei eine nachfolgende Reinigung überflüssig ist. Der Umsatz ist praktisch quantitativ, der Restaromatengehalt liegt vorzugsweise unter 0,01 Gew.-%, bezogen auf die gesamte Produktmenge. Das erhaltene Hydrierungsprodukt kann somit direkt einer Weiterverarbeitung zugeführt werden, ohne gereinigt werden zu müssen.

### LÖSUNGS- ODER VERDÜNNUNGSMITTEL

Beim erfindungsgemäßen Verfahren kann die Hydrierung unter Abwesenheit eines Lösungs- oder Verdünnungsmittels durchgeführt werden, d.h. gemäß einer Ausführungsform ist es nicht erforderlich, die Hydrierung in Lösung durchzuführen. Vorzugsweise wird jedoch ein Lösungs- oder Verdünnungsmittel im erfindungsgemäßen Verfahren eingesetzt. Als Lösungs- oder Verdünnungsmittel kann jedes geeignete Lösungs- oder Verdünnungsmittel eingesetzt werden. Die Auswahl ist dabei nicht kritisch, beispielsweise können gemäß einer Ausführungsform die Lösungs- oder Verdünnungsmittel auch geringe Mengen an Wasser enthalten. Beispiele geeigneter Lösungs- oder Verdünnungsmittel schließen ein geradkettige oder cyclische Ether, wie beispielsweise Tetrahydrofuran oder Dioxan, sowie aliphatische Alkohole, in denen der Alkylrest vorzugsweise 1 bis 10 Kohlenstoffatome, insbesondere 3 bis 6 Kohlenstoffatome, aufweist. Beispiele bevorzugt verwendbarer Alkohole sind i-Propanol, n-Butanol, i-Butanol und n-Hexanol. Gemische dieser oder anderer Lösungs- oder Verdünnungsmittel können ebenfalls verwendet werden. Das Lösungs- oder Verdünnungsmittel kann in geeigneten Mengen verwendet werden, wobei solche Mengen bevorzugt sind, die zu einer 10-bis 70 gew.-% igen Lösung der zur Hydrierung vorgesehenen Verbindungen führen.

Als Lösungsmittel kann auch besonders bevorzugt das bei der Hydrierung nach dem erfindungsgemäßen Verfahren gebildete Produkt eingesetzt werden, gegebenenfalls neben anderen Lösungs- oder Verdünnungsmitteln. In diesem Fall kann ein Teil des im Hydrierungsverfahren gebildeten Produktes den zu hydrierenden Verbindungen beigemischt werden. Bezogen auf das Gewicht der zur Hydrierung vorgesehenen Verbindungen wird vorzugsweise die 1- bis 30fache, besonders bevorzugt die 5- bis 20fache, insbesondere die 5- bis 10fache Menge des Hydrierungsproduktes als Lösungs- oder Verdünnungsmittel zugemischt.

### HYDRIERUNG

Die Hydrierung wird bei geeigneten Drücken und Temperaturen durchgeführt. Bevorzugt sind Drücke oberhalb von 50 bar, vorzugsweise von 150 bis 300 bar. Bevorzugte Temperaturen liegen in einem Bereich von 100 bis 270 °C, vorzugsweise 150 bis 220 °C.

Das Hydrierungsverfahren kann kontinuierlich oder in Art eines Batch-Verfahrens durchgeführt werden. Beim kontinuierlichen Verfahren kann dabei ein Teil des den Reaktor verlassenden Hydrierungsproduktes dem Reaktorzulauf vor dem Reaktor zugeführt werden. Dabei wird eine solche Menge an den Reaktor verlassendem Hydrierungsprodukt als Lösungsmittel rückgeführt, daß die vorstehend genannten Mengenverhältnisse erreicht werden. Die verbleibende Menge an Hydrierungsprodukt wird abgezogen.

Bei kontinuierlicher Prozeßführung kann dabei die Menge der zur Hydrierung vorgesehenen Verbindung bzw. Verbindungen vorzugsweise im Bereich von 0,05 bis 3 l pro Liter Katalysator pro Stunde betragen, vorzugsweise 0,1 bis 1 l pro Liter Katalysator pro Stunde.

Als Hydriergase können beliebige Gase verwendet werden, die freien Wasserstoff enthalten und keine schädlichen Mengen an Katalysatorgiften, wie beispielsweise CO, aufweisen. Beispielsweise können Reformerabgase verwendet werden. Vorzugsweise wird reiner Wasserstoff als Hydriergas verwendet.

Bei Verwendung des erfindungsgemäßen Hydrierkatalisators werden insbesondere bei der Hydrierung von 4-Alkyl- oder 4-Alkoxysubstituierten Phenolen, wie sie vorstehend beschrieben sind, überwiegend trans-cycloaliphatische Verbindungen erhalten. Der Anteil an trans-cycloaliphatischen Verbindungen beträgt dabei gemäß einer Ausführungsform der Erfindung mindestens 60 %, vorzugsweise mindestens 65 %.

Nachstehend wird die Erfindung anhand von Ausführungsbeispielen näher erläutert.

### BEISPIEL 1

### Herstellung des Katalysators

Ein makroporöser Aluminiumoxidträger in Form von 8 x 8 x 3 mm-Ringen, der eine Oberfläche von 1,75 m²/g, bestimmt nach dem BET-Verfahren, ein Porenvolumen von 0,531 ml/g sowie einen Porendurchmesser von 0,6 µm und 20 µm bei einer bimodalen Verteilung aufwies, wurde mit einer wäßrigen Ruthenium-(III)-nitrat-Lösung getränkt, die eine Konzentration von 0,7 bis 1 % Metall, bezogen auf das Gewicht der Lösung, aufwies. Das von Träger aufgenommene Lösungsvolumen entsprach dabei in etwa dem Porenvolumen des verwendeten Trägers. Sodann wurde der mit der Ruthenium(III)-nitrat-Lösung getränkte Träger bei 120 °C unter Bewegung getrocknet und bei 200 °C im Wasserstoffstrom reduziert. Der so hergestellte Katalysator enthält 0,5 Gew.-% Ruthenium, bezogen auf das Gesamtgewicht des Katalysators, und weist eine Rutheniumoberfläche von 0,76 m²/g auf, bestimmt durch H₂-Puls-Chemiesorption (Puls Chemiesorp 2700, 35 °C).

### BEISPIEL 2

3,2 l des nach Beispiel 1 hergestellten Katalysators, der 0,5 Gew.-% Ruthenium auf einem makroporösen Al₂O₃-Träger enthielt, wurden in einen elektrisch beheizten Durchflußreaktor eingefüllt, der mit einem Abscheider ausgestattet war. Sodann wurde ohne vorhergehende Aktivierung des Katalysators eine Hydrierung von p-tert.-Butylphenol durchgeführt bei 260 bar und 180 °C. Das p-tert.-Butylphenol wurde dabei als 50 gew.-%ige Lösung in THF dem Reaktor zugeführt. Stündlich wurden 2500 g dieser Lösung durch den Durchflußreaktor geleitet. Die Hydrierung wurde dabei kontinuierlich in Sumpffahrweise ausgeführt. Die kontinuierlich dem Reaktor zugeführte Menge an p-tert.-Butylphenol-Lösung in THF entsprach einer Katalysatorbelastung von ca. 400 g/l Katalysator x h (800 g der 50 Gew.-%igen Lösung). Nach Abdestillieren des Lösungsmittels hatte das Hydrierungsprodukt im Mittel die nachstehende Zusammensetzung (in Gew.-% des gesamten Hydrierungsproduktes):

| | |
|---|---|
| < 0,01 % | 4-tert.-Butylcyclohexan |
| 67,0% | trans-4-tert.-Butylcyclohexanol |
| 32,9% | cis-4-tert.-Butylcyclohexanol |
| < 0,01 % | p-tert.-Butylphenol |
| < 0,1 % | unbekannte Verbindungen |

### BEISPIEL 3

Es wurde eine Hydrierung wie in Beispiel 2 beschrieben durchgeführt, jedoch wurden stündlich 3500 g der 50 gew.-%igen Lösung von p-tert.-Butylphenol in THF durch den Reaktor geleitet. Die Temperatur betrug dabei 200 °C statt 180 °C. Nach Abdestillieren des Lösungsmittels hatte das Hydrierungsprodukt im Mittel die nachstehende Zusammensetzung:

| | |
|---|---|
| < 0,01 % | 4-tert.-Butylcyclohexan |
| 68,8% | trans-4-tert.-Butylcyclohexanol |
| 31,0% | cis-4-tert.-Butylcyclohexanol |
| < 0,01 % | p-tert.-Butylphenol |
| < 0,2% | unbekannte Verbindungen |

### BEISPIEL 4

Es wurde eine Hydrierung wie in Beispiel 2 beschrieben durchgeführt, jedoch wurde statt einer 50 gew.-%igen Lösung von p-tert.-Butylphenol in THF eine 50 gew.-%ige Lösung in i-Butanol eingesetzt.

Nach Abdestillieren des Lösungsmittels hatte das Hydrierungsprodukt im Mittel die nachstehende Zusammensetzung:

| | |
|---|---|
| < 0,01 % | 4-tert.-Butylcyclohexan |
| 67,5% | trans-4-tert. -Butylcyclohexanol |
| 32,4% | cis-4-tert.-Butylcyclohexanol |
| < 0,01 % | p-tert.-Butylphenol |
| < 0,1 % | unbekannte Verbindungen |

### BEISPIEL 5

500 ml des makroporösen Katalysators aus Beispiel 1 (0,5 Gew.-% Ru auf Al₂O₃) wurden in einem Katalysatorkorb eines 3,5 1 fassenden Druckautoklaven vorgelegt. 2 kg einer 50 gew.-%igen Lösung von Bisphenol A in THF wurden in den Autoklaven eingetragen.

Anschließend wurden 200 bar Wasserstoff aufgepreßt und bei einer Temperatur von 150 °C für 5 Stunden die Hydrierung durchgeführt.

Nach der Umsetzung wurde das Gemisch auf Raumtemperatur abgekühlt, auf Umgebungsdruck entspannt, und das Lösungsmittel wurde abdestilliert. Der Umsatz zum entsprechenden cycloaliphatischen Diol-Isomerengemisch war quantitativ, wobei der Restaromatengehalt kleiner als 0,01 Gew.-% war.

Die Versuchsergebnisse der kontinuierlichen oder diskontinuierlichen Hydrierung mit dem erfindungsgemäß verwendeten Katalysator zeigen, daß aromatische Verbindungen, in denen mindestens eine Hydroxylgruppe und ein ggf. substituierter C₁₋₁₀-Alkylrest an einen aromatischen Kern gebunden sind, zu den cycloaliphatischen Hydrierungsprodukten führt, wobei nur ein minimaler Anteil an Nebenprodukten entsteht. Bei der Hydrierung von p-tert.-Butylphenol wird überwiegend trans-4-tert.-Butylcyclohexanol gebildet, es wird dabei mindestens doppelt so viel der trans-Verbindung im Vergleich zur cis-Verbindung gebildet.

## Patentansprüche

1. Verfahren zur Hydrierung von aromatischen Verbindungen, in denen mindestens eine Hydroxylgruppe an einen aromatischen Kern gebunden ist, wobei man mindestens eine dieser Verbindungen in Gegenwart eines Katalysators mit freiem Wasserstoff in Berührung bringt, wobei der Katalysator Ruthenium und gegebenenfalls mindestens ein Metall der I-, VII- oder VIII-Nebengruppe in einer Menge von 0,01 bis 30 Gew.-%, bezogen auf das Gesamtgewicht des Katalysators, aufgebracht auf einen Träger, umfaßt, wobei der Träger einen mittleren Porendurchmesser von mindestens 0,1 µm, und eine Oberfläche von höchstens 15 m²/g, aufweist.

2. Verfahren nach Anspruch 1, wobei in den aromatischen Verbindungen außerdem mindestens ein gegebenenfalls substituierter C₁₋₁₀-Alkyl-und/oder Alkoxyrest an den aromatischen Kern gebunden ist.

3. Verfahren nach Anspruch 1, wobei der Katalysator einen oder mehrere der folgenden Merkmale aufweist:
- Die Porengrößenverteilung des Trägers ist annähernd bimodal;
- Der Träger ist ausgewählt aus Aktivkohle, Siliciumcarbid, Aluminiumoxid, Siliciumdioxid, Titandioxid, Zirkoniumdioxid, Magnesiumoxid, Zinkoxid oder deren Gemischen, vorzugsweise Aluminiumoxid oder Zirkoniumdioxid;
- Das mindestens eine Metall der I-, VII- oder VIII-Nebengruppe ist ausgewählt aus Kupfer, Rhenium, Kobalt und Nickel oder Gemischen davon;
- Das auf den Träger aufgebrachte Metall weist eine Oberfläche von 0,01 bis 10 m² pro g, des Katalysators auf.

4. Verfahren nach einem der vorstehenden Ansprüche, wobei der Katalysator aus einem makroporösen Aluminiumoxidträger mit einer Oberfläche von etwa 1,75 m²/g, einem Porenvolumen von etwa 0,531 ml/g, einer annähernd bimodalen Porengrößenverteilung mit Porendurchmessern von etwa 0,6 µm und etwa 20 µm besteht, auf den Ruthenium in einer Menge von etwa 0,5 Gew.-%, bezogen auf das Gesamtgewicht des Katalysators, aufgetragen ist, und wobei die Rutheniumoberfläche auf dem Träger etwa 0,76 m² pro g des Katalysators beträgt.

5. Verfahren nach einem der vorstehenden Ansprüche, wobei die aromatische Verbindung ein mit einem C₁₋₁₀-Alkylrest, substituiertes Phenol ist, wobei der Alkylrest gegebenenfalls substituiert ist mit einem aromatischen Rest.

6. Verfahren nach einem der vorstehenden Ansprüche, wobei die Hydrierung in Gegenwart eines Lösungs- oder Verdünnungsmittels durchgeführt wird.

7. Verfahren nach Anspruch 6, wobei als Lösungs- oder Verdünnungsmittel das Hydrierungsprodukt verwendet wird, das in einer 1 bis 30fachen Menge, bezogen auf das Gewicht der zu hydrierenden Verbindung, vorliegt.

## Claims

1. A process for hydrogenating aromatic compounds in which at least one hydroxyl group is bonded to an aromatic ring, which comprises bringing at least one of these compounds into contact with free hydrogen in the presence of a catalyst, wherein the catalyst comprises ruthenium and, if desired, at least one metal of transition groups I, VII and VIII in an amount of from 0.01 to 30% by weight, based on the total weight of the catalyst, applied to a support, where the support has a mean pore diameter of at least 0.1µm and a surface area of at most 15 m²/g.

2. A process as claimed in claim 1, wherein, in the aromatic compounds, at least one unsubstituted or substituted C₁-C₁₀-alkyl and/or alkoxy radical is additionally bonded to the aromatic ring.

3. A process as claimed in claim 1, wherein the catalyst has one or more of the following features:
- the pore size distribution of the support is approximately bimodal
- the support is selected from the group consisting of activated carbon, silicon carbide, aluminum oxide, silicon dioxide, titanium dioxide, zirconium dioxide, magnesium oxide, zinc oxide or mixtures thereof, preferably aluminum oxide or zirconium dioxide;
- the metal or metals of transit-ion groups I, VII and VIII is/are selected from the group consisting of copper, rhenium, cobalt and nickel or mixtures thereof;
- the metal applied to the support has a surface area of from 0.01 to 10 m² per g of the catalyst.

4. A process as claimed in any of the preceding claims, wherein the catalyst comprises a macroporous aluminum oxide support having a surface area of about 1.75 m²/g, a pore volume of about 0.531 ml/g and an approximately bimodal pore size distribution with pore diameters of about 0.6 µm and about 20 µm, on which ruthenium is applied in an amount of about 0.5% by weight, based on the total weight of the catalyst, and the ruthenium surface area on the support is about 0.76 m² per g of the catalyst.

5. A process as claimed in any of the preceding claims, wherein the aromatic compound is a phenol substituted by a C₁-C₁₀-alkyl radical, where the alkyl radical may be substituted by an aromatic radical.

6. A process as claimed in any of the preceding claims, wherein the hydrogenation is carried out in the presence of a solvent or diluent.

7. A process as claimed in claim 6, wherein the solvent or diluent used is the hydrogenation product which is present in an amount of from 1 to 30 times the weight of the compound to be hydrogenated.

## Revendications

1. Procédé d'hydrogénation de composés aromatiques, dans lesquels au moins un groupe hydroxyle est lié à un noyau aromatique, procédé dans lequel on met en contact au moins un de ces composés avec de l'hydrogène libre en présence d'un catalyseur, le catalyseur comportant, supporté sur un support, du ruthénium et éventuellement au moins un métal du groupe secondaire I, VII ou VIII en une quantité de 0,01 à 30% en poids, par rapport au poids total du catalyseur, le support présentant un diamètre de pores moyen d'au moins 0,1 µm, et une surface d'au maximum 15 m²/g.

2. Procédé suivant la revendication 1, dans lequel au moins un radical alcoxy et/ou alkyle en C₁-C₁₀ éventuellement substitué est en outre lié sur le noyau aromatique dans les composés aromatiques.

3. Procédé suivant la revendication 1, dans lequel le catalyseur présente une ou plusieurs des particularités suivantes :
- la répartition de la taille des pores du support est approximativement bimodale,
- le support est choisi parmi du charbon actif, du carbure de silicium, de l'oxyde d'aluminium, du dioxyde de silicium, du dioxyde de titane, du dioxyde de zirconium, de l'oxyde de magnésium, de l'oxyde de zinc ou leurs mélanges, de préférence de l'oxyde d'aluminium ou du dioxyde de zirconium,
- le au moins un métal du groupe secondaire I, VII ou VIII est choisi parmi du cuivre, du rhénium, du cobalt et du nickel ou leurs mélanges,
- le métal appliqué sur le support présente une surface de 0,01 à 10 m²/g du catalyseur.

4. Procédé suivant l'une des revendications précédentes, dans lequel le catalyseur est constitué d'un support d'oxyde d'aluminium macroporeux ayant une surface d'environ 1,75 m²/g, un volume poreux d'environ 0,531 ml/g, une répartition approximativement bimodale de la taille des pores avec des dimensions de pore d'environ 0,6 µm et environ 20 µm, support sur lequel du ruthénium est appliqué en une quantité d'environ 0,5% en poids par rapport au poids total du catalyseur, et dans lequel la surface du ruthénium sur le support est d'environ 0,76 m²/g du catalyseur.

5. Procédé suivant l'une des revendications précédentes, dans lequel le composé aromatique est un phénol substitué par un radical alkyle en C₁-C₁₀, le radical alkyle étant éventuellement substitué par un radical aromatique.

6. Procédé suivant l'une des revendications précédentes, dans lequel l'hydrogénation est effectuée en présence d'un solvant ou d'un diluant.

7. Procédé suivant la revendication 6, dans lequel on utilise, comme solvant ou diluant, le produit d'hydrogénation qui se présente en une quantité de 1 à 30 fois, par rapport au poids du composé à hydrogéner.
